# EUROPEAN PATENT APPLICATION

(11) **EP 3 591 662 A1**
(43) Date of publication of application: **08.01.2020**
(21) Application number: 18182068.9
(22) Date of filing: 05.07.2018
(51) Int. Cl.: G16H 40/60, A61M 5/172, G06F 21/44

(54) **MEDICAL DEVICE AND SECURE CONTROL SYSTEM**

(71) Applicant: Advanced Microfluidics SA, 1024 Ecublens (CH)
(72) Inventor: KLOPFENSTEIN, Denis, 1110 Morges (CH); FRIDEZ, Pierre, 1055 Froideville (CH)
(74) Representative: reuteler & cie SA

(57) **Abstract**

**Medical device and secure control system** (1) comprising a wearable medical device (2) and a portable medical device controller (4) for control of the medical device. The medical device (2) comprises a control system comprising a communications module including at least short range wireless communications capability. The medical device controller (4) comprises a communications module (17) including at least a short range wireless communications capability for communication with the medical device (2) and a consumer electronic device (12) including any one or more of a smartphone, a personal computer, and a computer tablet. The medical device controller (4) further comprises a memory (14) storing a computer program and encryption keys configured to establish an encrypted communication with the medical device (2) and with the consumer electronics device, and a processor (15) configured to execute the computer program. The medical device controller (4), the consumer electronic device (12) and the medical device store each at least one journal (16) for exchange of journal entries between the medical device controller (4) and the consumer electronic device and between the medical device controller (4) and the medical device (2).

## Description

### Field of the Invention

This invention relates to a medical device and a system for the control of the medical device. One aspect of this invention relates in particular to a system for the control of a drug delivery device. A field of application of the drug delivery device includes the administration of a liquid drug via an infusion set or a patch device.

### Background of the Invention

One of the fields addressed by the present invention relates to the administration of insulin to patients suffering from diabetes.

Diabetes mellitus is the second more frequent disease in the world and the cause of an increasing number of deaths each year. It has more than 8.5% of prevalence in adult population, which means around 415 Million people were suffering it in 2015, subjected to further complications and risks associated with the disease, and a substantial increase is predicted by 2040: around 642 Million people will be affected. Therefore, about 12% of global health expenditures are spent on diabetes treatment (about $673 billions). This disease has been increasing in the last decade not only in developed countries like US and Europe, but also in middle- and low-income countries and this trend is expected to continue and become worse in the upcoming years. The main causes are hereditary, dietary changes, bad health habits and an excessive use of sugar in the food industry (all related to obesity), along with an extended human lifespan.

Type I diabetes patients, mostly, depend on insulin treatment to manage their symptoms and to avoid further complications such as heart and kidney disease, blindness or limb problems. The most common delivery systems are insulin syringes, pens or jet injectors, but they do not allow to stabilize accurately the glucose level, are prone to manipulation errors and are dependent on medical support. Accuracy errors can cause over- or under-insulin dosing, leading to health complications such as hypoglycemia, coma, hyperglycemia, ketoacidosis or even death. They are also responsible for higher expenditures to the healthcare system (33% of medical errors in this domain are insulin delivery-related). In addition, insulin syringes, pens or jet cannot deliver insulin continuously thus are not able to stabilize accurately the glucose level which is a major drawback for long term patient's health. The need for easier-to-handle, more accurate insulin delivery devices has led to the development of various insulin pumps that are carried by patients and that can deliver both basal rate and bolus injections of insulin. Existing insulin delivery devices include portable devices that may be carried on a patient's clothing and connected via a catheter to a patch needle (also known as an infusion set) with a transcutaneous cannula extending through the patient's skin. Also known are portable patch devices mounted directly against the patient's skin with a cannula extending through the patient's skin. In the latter case, a separate dedicated control device with a user interface is provided to wirelessly control the portable patch device. Such devices make insulin injection therapy relatively discreet and replace the need for frequent injections.

Despite their presence in the market, only around 1% of diabetes patients currently use an insulin pump and there are still many unresolved needs for diabetes patients and health care professionals.

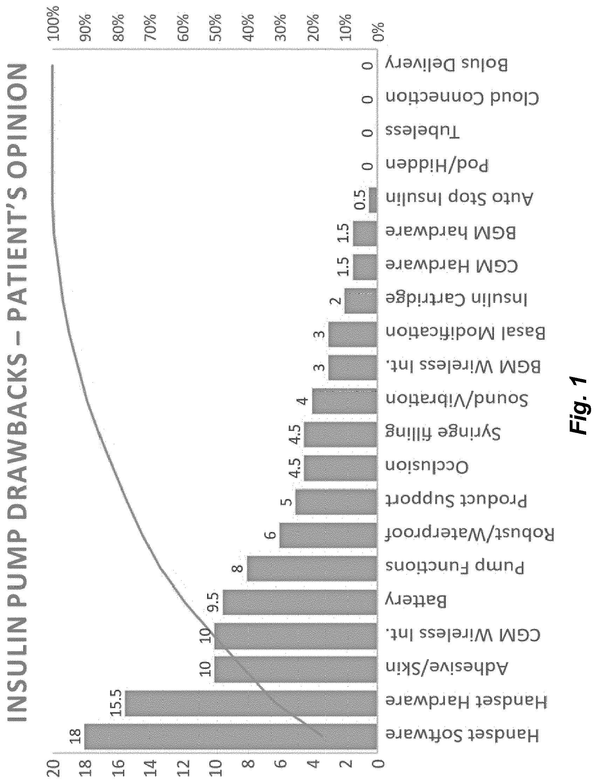

The inventors have realized that one of the important drawbacks of conventional drug delivery systems stems from the custom handset software and hardware of the control device, in particular the less ergonomic user interface of these devices compared to many widespread consumer devices such as smartphones which users are very familiar with. A small survey carried out by the inventors, as shown in figure 1, illustrates the main drawbacks the surveyed patients expressed in relation to existing insulin patch pumps.

Another drawback of conventional insulin pumps is the inability for caregivers to intervene remotely if needed, and more generally the difficulty in allowing various non-professional care givers such as a patient's parent or relative to assist in the control of drug delivery when needed (for instance for patients that are children or elderly persons). Type 1 diabetes patients over 65 years old represent more than 22% of the people who have diabetes. In addition, it is not rare for children below 14 to be diabetics and to use an insulin pump. For these specific populations, as well as for other users, a responsible caregiver is often mandatory. There is therefore a need for systems that allow easier control of medical devices, and in some circumstances there would be an advantage in allowing medical devices to be controlled remotely.

Improved usability and easy control of drug delivery and other medical devices would thus be advantageous to patients and care givers, provided that the safety of the device is not compromised. Medical devices require a high level of safety and software embedded therein has to follow very strict regulatory rules, mainly the international standard *IEC 62304* - *medical device software* - *software life cycle processes* which specifies life cycle requirements for the development of medical software and software within medical devices. It is harmonized by the European Union and the United States, and therefore can be used as a benchmark to comply with regulatory requirements from both these markets. Both European and US regulations distinguish three different categories of medical device software. The software safety classes according to IEC 62304 are defined as follows:
Class A: the software system cannot contribute to a hazardous situation or the software system can contribute to a hazardous situation which does not result in unacceptable risk after consideration of risk control measures external too the software system;
Class B: the software system can contribute to a hazardous situation which results in unacceptable risk after consideration of risk control measures external to the software system and the resulting possible harm is non-serious injury; and Class C: the software system can contribute to a hazardous situation which results in unacceptable risk after consideration of risk control measures external to the software system and the resulting possible harm is death or serious injury.

The medical device hardware must also comply with the EN 60601-1 and EN 60601-1-2 standards of the Medical Device Directive for the safety and electromagnetic compatibility, such as: immunity to electro-static-discharge; falling test; protection again ingress of liquid; ageing test; and Failure Mode Effects Analysis (FMEA). These criteria applied in relation with the definition of the *"Essential Performance"* for the medical device to be safe and efficient.Consumer electronic devices do not comply with such requirement, and anyway, none of these tests can be done on these devices.

This is one of the reasons that consumer electronics devices such as personal computers, smart phone, computer tablets and smart watches are not used to control medical devices. Such devices however have a high performance in terms of processing capability and the quality of the user interface, in particular the graphical user interface. Also, consumer electronic devices have software that is regularly updated to follow improvements and changes in communications technology, operating systems and display technology.

Implementation of electronics with the performance of consumer devices in medical devices is very costly in view of the much smaller volume of manufacturing units compared to the consumer market. Moreover, the need for multiple devices is generally undesirable for many users that prefer being able to perform many tasks with just a single device such as their smartphone.

The above considerations apply to drug administration devices for other drugs and diseases and more generally to many other medical devices that are controlled with custom control devices having a user interface for the entry of commands or the display of information.

### Summary of the Invention

In view of the foregoing, it is an object of this invention to provide a medical device and secure control system therefor that is safe and reliable, yet easy to use.

It would be advantageous to provide a medical device and secure control system therefor which allows the device to have a high autonomy (low power consumption) and is easy to carry and comfortable to wear.

It would be advantageous to provide a medical device and secure control system therefor which is cost effective to manufacture and use.

It would be advantageous to provide a medical device and secure control system therefor which allows non-professional care givers to manage the system at distance (telemedicine).

It would be advantageous to provide a medical device and secure control system therefor which facilitates treatment of a patient by health care professionals on site or remotely, and that improves access to patient's treatment and device use history.

It would be advantageous to provide a medical device and secure control system therefor which facilitates assistance provided by non-professional health care givers to patients.

It would be advantageous to provide a medical device and secure control system therefor which allows the medical device to be controlled by consumer electronic devices whose software thereof may only comply with class A or B requirements

It would be advantageous to provide a medical device and secure control system therefor which is configured to easily add a new consumer electronic device while taking into consideration the hardware limitation of the consumer electronic device in term of reliability.

Objects of this invention have been achieved by providing a method of operating a medical device according to claim 1.

Objects of this invention have been achieved by providing a method of initialization of a medical device according to claim 8.

Disclosed herein, according to a first aspect of the invention, is a method of operating a medical device and secure control system. The medical device and secure control system includes a medical device and a medical device controller comprising a communications module including at least a short range wireless communications capability for communication with one or a plurality of consumer electronic devices including any one of a smartphone, a personal computer, a smartwatch and a computer tablet. Each of the medical device and the medical device controller comprises a memory storing one or a plurality of journals for exchange of journal entries between the medical device controller and the medical device, and between said medical device controller and said one or a plurality of consumer electronic devices. The method comprises: **a)** receiving in the medical device controller pairing parameters from a consumer electronic device; **b)** comparing these pairing parameters with pairing parameters stored in the memory of the medical device controller during a pairing operation; **c)** receiving in the medical device controller a journal entries history of said consumer electronic device in case of a successful pairing operation; **d)** comparing the journal entries history received from said consumer electronic device with the journal entries history stored in the memory of the medical device controller; **e)** validating a session between said consumer electronic device and the medical device controller for data exchange if the journal entries history of said consumer electronic device matches the journal entries history of the medical device controller.

In an advantageous embodiment, the method further comprises writing a journal entry in the at least one journal of the medical device, in the at least one journal of the medical device, and in the at least one journal of said consumer electronic device, through a communications network such as an internet of thing network, wherein the journal entry is indicative of the type of data exchange under step **e).**

In an advantageous embodiment, the medical device controller is configured for communication with at least a first and a second consumer electronic device. The method further comprises writing a journal entry in the at least one journal of the medical device, in the at least one journal of the medical device, in the at least one journal of said first consumer electronic device, and in the at least one journal of said second consumer electronic device.

In an advantageous embodiment, once the session under step **e)** has been validated, the method performs the following steps:
- sending instructions from the medical device controller to said consumer electronic device to display an image with one or more entry field for data input; and
- receiving in the medical device controller a copy of the image or at least a portion of the image, sent by said consumer electronic device, comprising data input for operation of the medical device.

In an advantageous embodiment, said image or said at least a portion of the image contains pixels that constitute an identification code which is read by the medical device controller upon receipt of the image so as to determine whether the image displayed on said consumer electronic device corresponds to the image sent from said consumer electronic device to the medical device controller in order to detect a spoofed or hacked transmission of information.

In an advantageous embodiment, the method further comprises: receiving in the medical device controller pairing parameters from the medical device; comparing these pairing parameters with pairing parameters stored in the memory of the medical device controller during a pairing operation; receiving in the medical device controller a journal entries history of the medical device in case of a successful pairing operation; comparing the journal entries history of the medical device with the journal entries history stored in the memory of the medical device controller; validating a session between the medical device and the medical device controller if the journal entries history of the medical device matches the journal entries history of the medical device controller, and operating the medical device based on said data input.

In an advantageous embodiment, the medical device controller comprises journal entries containing user identification, user rights, and operations instructed to the medical device in order to assign different rights to a user for operating the medical device according to his/her profile.

In an advantageous embodiment, the rights are configurable by a medical practitioner.

In an advantageous embodiment, the medical device controller comprises journal entries containing safeguards parameters configurable by a medical practitioner so as to ensure that the user cannot perform certain functions which may have life-threatening consequences. Safeguards parameters may prevent for example a user to enter a bolus value for insulin injection which is outside a specific range.

In an embodiment, the medical device controller is incorporated or physically connected to the medical device.

In an embodiment, the medical device controller is formed as a separate component from the medical device, wherein the communications module includes at least a short range wireless communications capability for communication with the medical device.

Disclosed herein, according to a second aspect of the invention, is a method of initialization of a medical device and secure control system comprising a medical device and a medical device controller. The medical device controller comprises a communications module including at least a short range wireless communications capability for communication with a medical device and a consumer electronic device including any one or more of a smartphone, a personal computer, a smartwatch and a computer tablet. The medical device controller further comprises a memory and a processor. The method comprises: installing an operating system software in the medical device controller for operation thereof; storing pairing parameters in a pairing journal created in the memory of the medical device controller; and storing journal entries about a medical practitioner that will be responsible for the creation of further journal entries that will link a consumer electronic device with the medical device controller.

In an advantageous embodiment, pairing parameters are first entered in a pairing journal stored in a medical data server. A copy of the pairing journal is then stored in the memory of the medical device controller through a communications network.

In an advantageous embodiment, adding a user to the medical device controller and assigning particular rights to the user for particular actions the user may perform with his consumer electronic device require the following steps: a medical practitioner uses an electronic interface, for example a computer, to open a session with the medical data server and to select in a user journal a user to be added in the medical device controller; a pairing operation is performed between the medical device controller and the medical data server, through a communication network, during which pairing parameters are compared for identification of the medical device controller; the user downloads a software application on his consumer electronic device and uses the camera thereof to capture an identification code on the medical device controller, which may for instance be in the form of a QR code; the identification code is entered by the medical practitioner on the electronic interface and sent to the medical data server; the medical practitioner enters one or more journal entries in the user journal stored in the medical data server in order to assign to the user different rights for particular actions the user may perform with his consumer electronic device; and a copy of the user journal is stored in the memory of the medical device controller through the communications network.

Disclosed herein, according to a third aspect of the invention, is a medical device and secure control system comprising a wearable medical device and a medical device configured for control of the medical device. The medical device controller comprises a communications module including at least a short range wireless communications capability for communication with a consumer electronic device including any one or more of a smartphone, a personal computer, a smartwatch and a computer tablet. The medical device controller further comprises a memory storing a computer program and encryption keys configured to establish an encrypted communication with the consumer electronics device, and a processor configured to execute the computer program. The medical device controller and the medical device store each at least one journal for exchange of journal entries between the medical device controller and the consumer electronic device and between the medical device controller and the medical device. The journal entries comprise user identification, user rights, and operations instructed to the medical device. The medical device controller is configured to transmit via said encrypted communication: i) a data entry image for presentation on a graphical display of the consumer electronic device, or ii) instructions to upload an entry image stored in the memory of the consumer electronic device, and to receive via said encrypted communication an instruction for operation of the medical device. The medical device controller is configured to transmit said instruction for operation of the medical device to the medical device.

In an embodiment, the medical device controller is a portable medical device controller formed as a separate component from the wearable medical device and intended to be carried separately in short range wireless communication with the wearable medical device. The medical device comprises a control system comprising a communications module including at least short range wireless communications capability. The communications module of the medical device controller includes at least a short range wireless communications capability for communication with the medical device.

In an embodiment, the medical device controller is incorporated or physically connected to the medical device.

In an advantageous embodiment, the at least one journal stored in the medical device controller, the consumer electronic device and the medical device comprise each past instructions (hereafter journal entries history) exchanged between the medical device controller and the consumer electronic device and between the medical device controller and the medical device. The medical device controller is configured to verify the integrity of the consumer electronic device based on the comparison of both journal entries histories stored respectively in the medical device controller and the consumer electronic device.

In an advantageous embodiment, a login session between the consumer electronic device and the medical device controller is allowed only if both journal histories are identical.

In an advantageous embodiment, the medical device controller is configured to incrementally write an entry in the at least one journal stored in its memory for each login session with the consumer electronic device and with the medical device. The medical device controller is further configured to copy said entry in the at least one journal stored in the memory of the consumer electronic device and in the memory of the medical device.

In an advantageous embodiment, the consumer electronic device is configured to execute a software application for displaying different user-interface templates or messages according to an output signal of the medical device controller.

In an advantageous embodiment, the medical device and secure control system further comprises a medical data server storing a copy of the at least one journal stored in the medical device controller, and secure communications between the medical data server and the medical device controller. The medical device controller is configured to update said copy through said secure communications.

In an advantageous embodiment, the medical data server is configured to verify the integrity of the at least one journal stored in the consumer electronic device when the medical device controller is not available.

In an advantageous embodiment, the consumer electronic device is connected to the medical device controller through a pairing operation which compares pairing parameters of the consumer electronic device and the medical device controller which have been saved in their respective memory during an initialization step.

In an advantageous embodiment, the medical device is a drug delivery system, preferably a patch pump.

In an advantageous embodiment, the processor of the medical device controller is configured to execute the computer program in order to assign the role of the consumer electronic device to limited functions so as to comply with the software requirements of classes A or B.

In an advantageous embodiment, the role of said consumer electronic device is limited to the following functions: display of images in the form of user-interface templates with one or more data entry fields for user input; display of images containing messages; and data encryption and data transfer for secure communications.

In an advantageous embodiment, the operation of the medical device is based on data inputs in one or more data entry fields of at least one user-interface template displayed by the consumer electronic device.

Disclosed herein, according to a fourth aspect of the invention, is software application for the consumer electronic device of the medical device and secure control system according to the third aspect of the invention and any embodiment thereof disclosed above. The software application is configured to store data of a catalogue of images in the memory of said consumer electronic device when the software application is installed thereon. The images represent different user-interface templates to be displayed on the consumer electronic device according to the instructions received from the medical device controller in order to provide data entry fields allowing parameters to be entered. The software application performs the following operations when executed: selecting a user-interface template according to instructions received from the medical device controller; displaying on the consumer electronic device said user-interface template for data entry; capturing at least a portion of the displayed image on the consumer electronic device with the relevant parameters once entered; and sending the captured portion of the displayed image to the medical device controller.

In an advantageous embodiment, the software application is configured to alter the image to be displayed on the consumer electronic device according to instructions received from the medical device controller.

Further objects and advantageous aspects of the invention will be apparent from the claims, and from the following detailed description and accompanying figures.

### Brief Description of the drawings

Figure 1 is a graph depicting the main drawbacks for type I diabetes for patients using insulin pump;
Figure 2 is a schematic illustration of the medical device and secure control system according to an embodiment of the invention;
Figure 3 is a schematic illustration of the medical device and secure control system according to an embodiment of the invention;
Figure 4 is a schematic illustration of the medical device and secure control system according to an embodiment of the invention;
Figure 5 is a schematic illustration of the medical device and secure control system according to an embodiment of the invention;
Figure 6 is a schematic illustration of the medical device and secure control system according to an embodiment of the invention;
Figure 7 is a schematic illustration of the medical device and secure control system according to an embodiment of the invention;
Figure 8 is a schematic illustration of the medical device and secure control system according to an embodiment of the invention;
Figure 9 is a schematic illustration of the medical device and secure control system according to an embodiment of the invention;
Figure 10 is a schematic illustration of the medical device and secure control system according to an embodiment of the invention;
Figure 11 is a schematic illustration of the medical device and secure control system according to an embodiment of the invention; and
Figure 12 is a basic block diagram of the elements of the medical device controller of the medical device and secure control system.

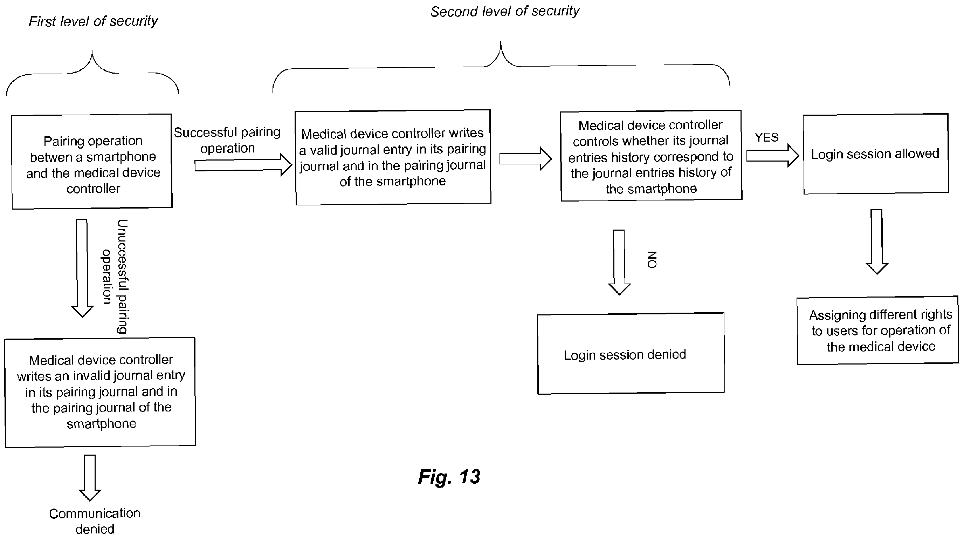

Figure 13 is a flow chart for validating a session for data exchange between the medical device controller and the consumer electronic device, and

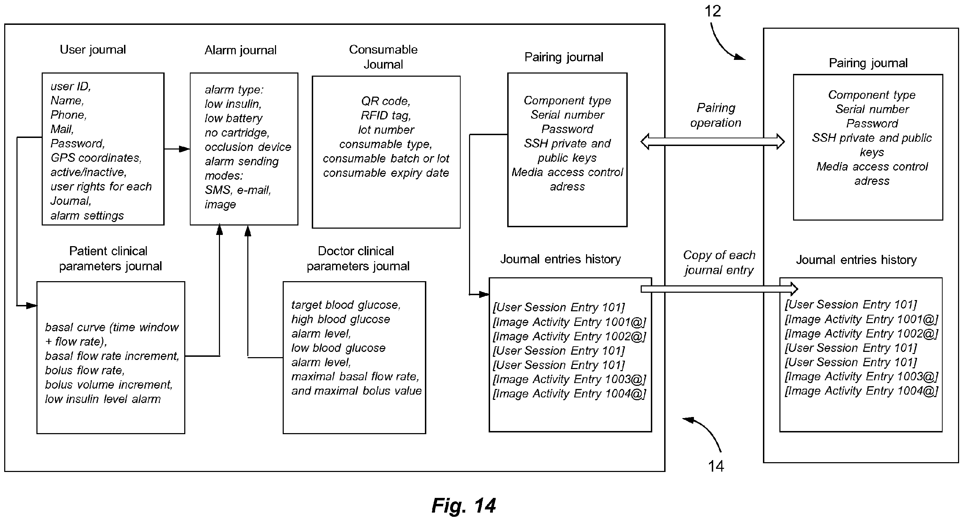

Fig. 14 is a schematic illustration of a pairing operation between the medical device controller and the consumer electronic device and an example of journals stored in the medical device controller.

### Detailed description of embodiments of the invention

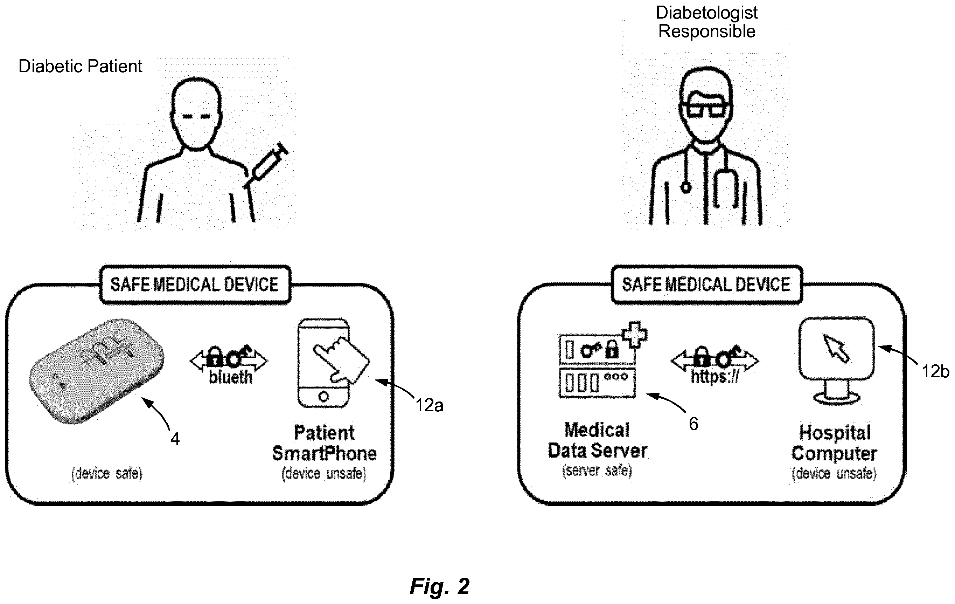

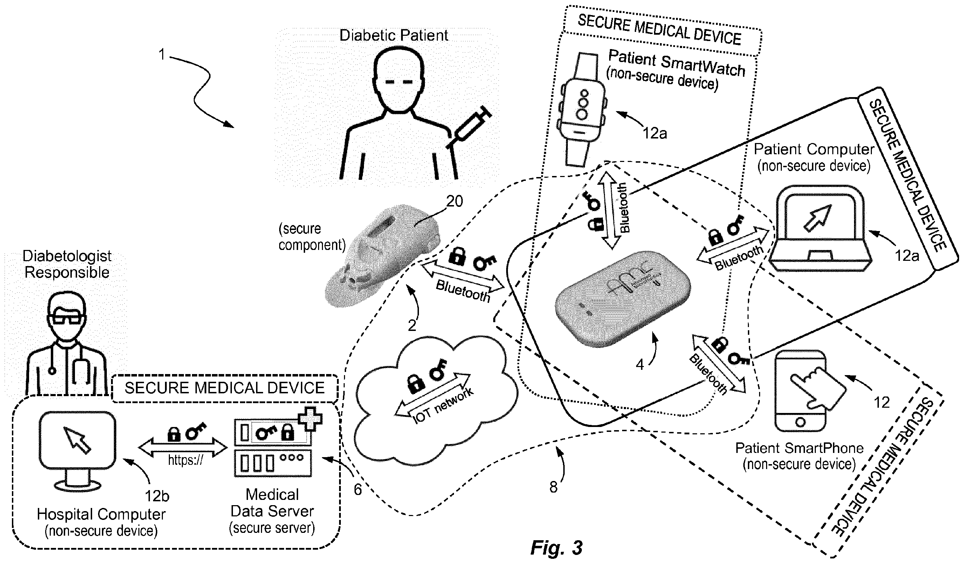

Referring to the figure 2 and 3, a medical device and secure control system 1 comprises a medical device 2 and a medical device controller 4.

According to an aspect of the invention, the medical device 2 is in a form of a portable handheld or wearable drug delivery device and in particular a drug delivery device for the administration of a liquid drug to a patient in need thereof. In an embodiment of the invention, the liquid drug is or includes insulin, for the treatment of diabetes.

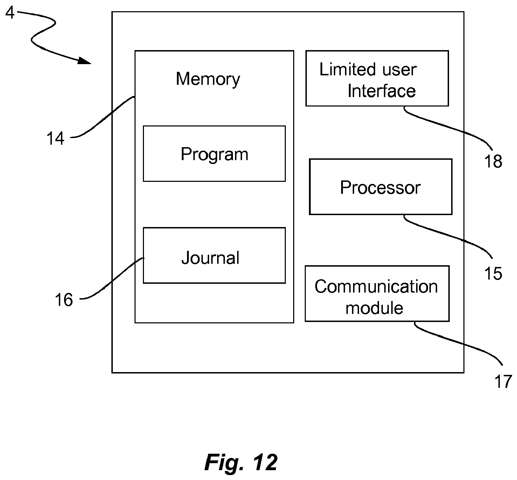

In an embodiment, the medical device controller 4 is a separate unit from the medical device and is configured to communicate in a wireless manner with the medical device 2 and may be carried by a patient for instance in a pocket of the patient's clothing or coupled to a belt or other article of clothing. The medical device controller 4 is configured to be carried in proximity to the medical device 2, typically on the patient wearing the medical device or within a few meters (e.g. up to 10m) to enable short range communication by means of a standard protocol for short range communications such as Bluetooth, between the medical device 2 and medical device controller 4. As shown in figure 12, the medical device controller 4 comprises a memory 14 storing a computer program, one or a plurality of journals 16, and a communications module 17 including at least a short range wireless communications capability for exchange of journal entries between the medical device controller 4 and a consumer electronic device 12 including any one or more of a smartphone, a personal computer, a smartwatch and a computer tablet.

In an embodiment, the medical device controller 4 is directly integrated in the medical device 2 and comprises a communications module 17 including at least a short range wireless communications capability for exchange of journal entries between the medical device controller 4 and one or more of the aforementioned consumer electronic device. Communication for exchange of journal entries between the medical device controller 4 and the medical device 2 for operation of the medical device may be achieved through electrical wiring.

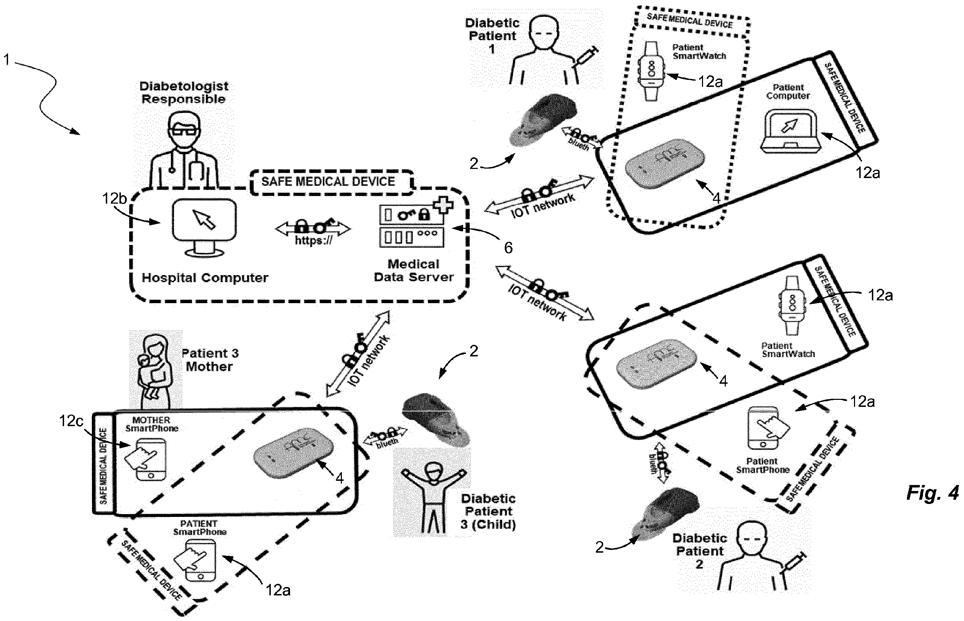

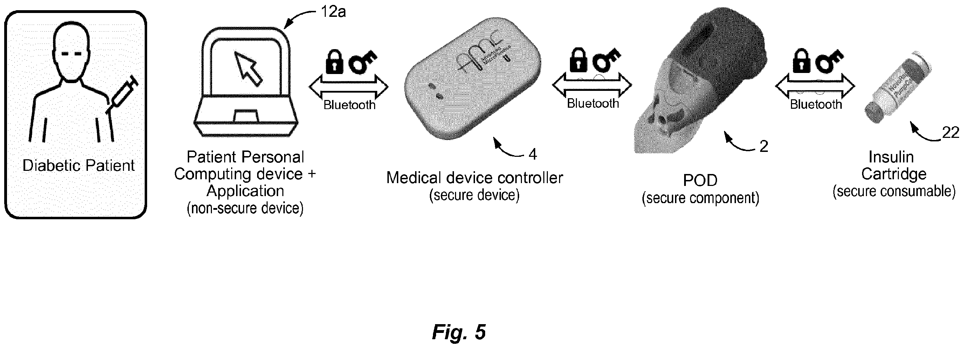

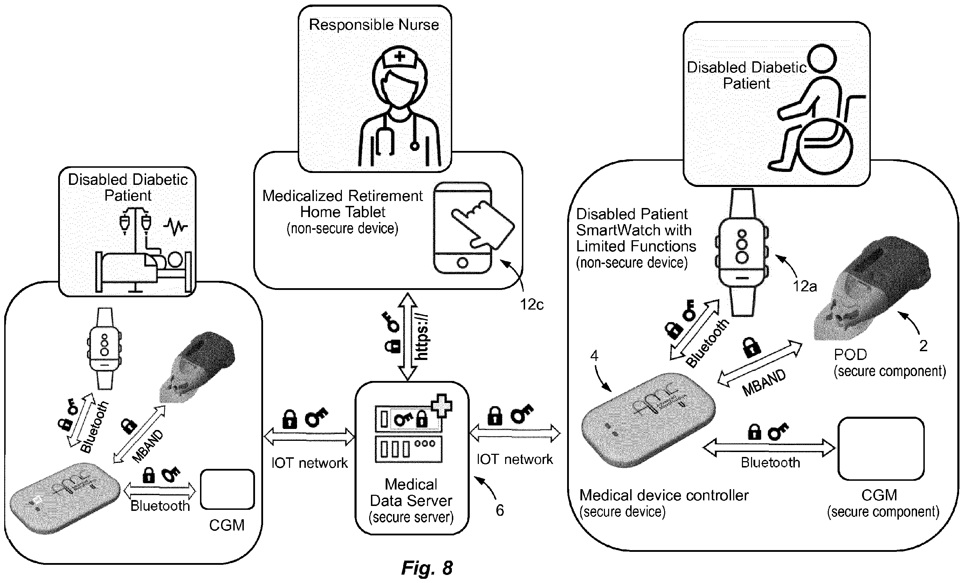

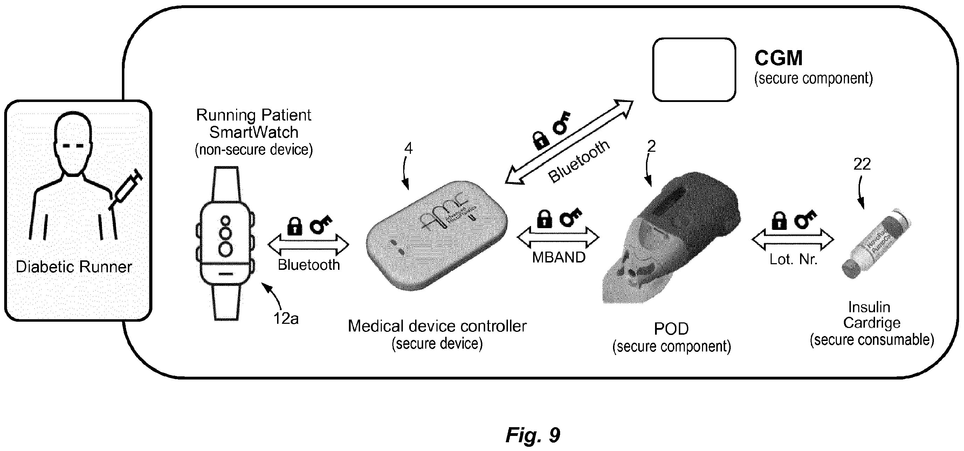

The medical device and secure control system 1 may have a multitude of configurations according to different embodiments as shown for example in figures 4 and 8, whereby the condition of several patients may managed through telemedicine by a single medical practitioner, or as shown for example in figures 5 and 9, whereby the patient may use only the medical device 2, the medical device controller and his consumer electronic device for insulin injection. Depending on the configuration of the medical device and secure control system 1, external electronic devices may for example comprise any one or more of a consumer electronic device 12a of a patient (hereinafter also called patient's electronic device), a consumer electronic device 12b of a doctor (hereinafter also called doctor's patient electronic device), a consumer electronic device 12c of a patient's friend or family member, the medical device 2 and a medical data server 6.

Referring to figure 12, the medical device controller 4 further comprises a processing unit 15 configured to execute the computer program, a communication module 17 and a limited user interface 18. The limited user interface 18 may simply comprise lights indicating the status of the medical device controller 4 or a simple screen indicating a few status indications of importance such as on/off status, battery charge status, and communication status, in particular whether the medical device controller 4 has established a communication link with the drug delivery device or with an external device. The communication link may for instance be via a short range connection such as Bluetooth, or an internet connection for internet of things (IOT) communication.

The medical device controller 4 however does not require a graphical user interface in the form of a screen as found on consumer electronic devices such as smartphones or personal computers. The medical device controller 4 serves to establish secure communication with the drug delivery device 2 and with external devices and ensures secure communications between external devices and the drug delivery device.

The medical device controller 4 is further configured to control operation of the drug delivery device based on inputs from the patient or another person authorized to enter instructions for the medical device 2. Persons that may interact with the drug delivery device include the patient, a healthcare professional, in particular a medical practitioner or a nurse, and in certain circumstances a non-professional care giver. The operations that the authorized user may instruct, or the information that the authorized user may access, depends on the rights given to the user that are defined by the type of user, their role and their associated rights that are stored in the memory of the medical device controller, in particular in a User journal.
Control of the medical device 2 and access to information in the medical device 2 may be performed solely through the medical device controller 4 or in conjunction with the medical device controller 4.

Within the scope of the invention, certain limited instructions may be received by the medical device 2 directly via a communication module of its control system, such situations including in particular emergency situations requiring for instance switching off of the medical device.

The medical device 2 and medical device controller 4 are secure devices because the hardware and software contained therein comprise dedicated application specific components and modules manufactured under control specifically for the purpose of a drug administration and in view of the regulatory requirements responding to the standards required for medical devices. For a medical device such as an insulin delivery system, the software should satisfy class C standard. Also, the operating system of these devices are application specific and the range of possible operations are limited compared to consumer electronic devices, and thus may be easily configured by the manufacture to be very resistant to external hacking or spoofing events.

The medical device controller 4 is configured via its communications module 17 to communicate with external devices 12 that are not application specific medical devices that may include a personal computing device such as a smartphone, portable computer, computer tablet or other consumer electronic devices comprising a graphical user interface allowing the user to view and enter information into the personal computing device. Typically, devices already carried around and widespreadly used by people are smartphones that have a user interface in the form of a screen, in particular a touch screen in which the user can view and enter information and instructions.

A software application is installed in the consumer electronic devices that allows communication with the medical device controller 4. The software application may be downloaded from a server through the internet or alternatively downloaded from the medical device controller 4 depending on the configuration. Advantageously, the application is configured to store data of a catalogue of images in the memory of the consumer electronic device 12 when the software application is installed thereon. The images may represent different user-interface templates or messages to be displayed on the screen of the consumer electronic device according to the instructions received from the medical device controller 4 in order to provide data entry fields allowing parameters to be entered. The medical device controller 4 may communicate with external devices once a pairing operation has been executed in which an encrypted communication channel between the medical device controller 4 and an external device is established. The communication channel may be via a short range communication protocol such as Bluetooth when the personal computing device is in the vicinity of the medical device controller 4, or may be established via the internet for example via an internet of things (IOT) network to a remote computing device, for instance the computing device 12b of a medical doctor or a medical data server 6 of a professional or healthcare institution. The software application installed on the patient's electronic device 12a, enables the patient to control the medical device 2 using for example his own smartphone and to access information, for instance drug delivery history or sensor measurements (e.g. blood glucose measurement if the drug delivery device comprises a blood glucose sensor), or other sensor information depending on the type of medical device.

A consumer electronic device 12 is considered as a non-secure device in a sense that the hardware and software implemented in the consumer electronic device does not respond to the safety requirements for a medical device.

According to the invention however, the consumer electronic device in conjunction with the medical device controller 4 and the medical device 2 form a secure system controlled by the medical device controller 4. The medical device controller 4 is configured to delegate tasks to external devices 12 or other devices that may be considered *per se* non-secure, in a manner that renders them secure, on the one hand by delegating tasks of a lower safety level and on the other hand by encrypting communications.

The tasks which require a high safety level are managed by the medical device controller 4. These tasks may be for example: management of the data necessary to operate the medical device 2; calculations (software algorithms) that determine output values (insulin dose) from input values (glucose values, dose limit defined by the doctor, pump parameters); alarm management, including alarm setting criteria, alarm transmission, alarm acknowledgement; all the steps requiring user inputs in order to start a clinical activity: verifying the user role and pairing, providing information, requiring information, requesting confirmation, requesting confirmation from another user; and management of the transmission of data, control of integrity, acknowledgement of data transmission.

The medical device controller 4 is in particular configured to assign the roles of consumer electronic devices 12 to functions of low level concern in terms of risk of injury, in order to comply with the software requirements of classes A or B of the international standard IEC 62304 for medical device software. The roles of each consumer electronic device 12 are mainly limited to the following functions: user-interface for data input; data encryption and data transfers; and management of journal entries in journals stored in the memory of each consumer electronic device 12. The medical device controller 4 may further contain threshold values stored in the memory, the threshold values serving to limit the range of operations that may be executed and instructed for operation of the medical device. For instance, a maximum value for a bolus injection of insulin may be stored so that any dosage instructions exceeding the threshold are limited to the threshold value or a warning is sent to the patient

In an embodiment, one feature of the secure communication comprises the transmission of the journal stored in the medical device controller to the external devices 12 that are paired to the medical device controller 4. Each external device that enters into communication with the medical device controller 4 comprises a copy of the journal. The history of operations and integrity of the journal may thus be verified at each selected step or at regular intervals by the medical device controller 4.

According to an embodiment of the invention, entry of an instruction to the medical device 2 by the patient using for example his smartphone may be performed as follows. The patient's electronic device 12a first needs to be paired with the medical device controller 4. In an embodiment, the medical device controller 4 advantageously manages and stores in its' memory the encryption keys for secure communication between paired devices. This allows pairing to be effected without access to a secure server and strengthens security in view of the limited access to the medical device controller application as well as its application specific software and hardware. After pairing of the patient's electronic device 12 with the medical device controller 4 via a secure (encrypted) short range communication, for instance an encrypted Bluetooth communication, the software application on the patient's electronic device sends a request to the medical device controller 4.

The medical device controller 4 transmits a navigation menu to the patient's electronic device 12 for display of the navigation menu on its screen. The navigation menu may provide data entry fields allowing the patient to enter values or operating instructions for the drug delivery device. These operating instructions may for instance constitute instructions to inject a bolus administration of insulin.

A screen capture of the instructions entered by the patient on his smartphone 12a is transmitted to the medical device controller 4. Alternatively, a portion of the screen containing the relevant information may be transmitted to the medical device controller 4.

The initial navigation menu sent by the medical device controller 4 as an image to the patient's electronic device 12a may further include pixels that constitute an identification code to ensure that the image returned from the patient's electronic device 12a to the medical device controller 4 corresponds to the navigation menu image received by the patient electronic device 12a. In view of the screen capture transmitted to the medical device controller 4 and the identification or control pixels contained in the image, a spoofed or hacked transmission of information can be detected.

The medical device controller 4 may further comprise parameters stored in its' memory that determine the range of instructions and amounts it may accept from an external device 12 depending on the type of User and associated role and rights stored in the journal. The medical device controller may further comprise threshold values for administration of a drug for instance the amount of insulin that may be injected at one time or over a certain period of time may be limited to a certain value that overrides any instructions going beyond the threshold values. Such threshold values set a limit of the possible dosage of the drug for the safety of the patient.

Advantageously, the patient may enter instructions for control of the medical device 2, or enter instructions to access information such as the history of administration for a certain period, without compromising the security and safety of the medical device system. Authorized doctors and other healthcare professionals may access information regarding usage of the medical device via the medical device controller 4 to verify treatment adherence, or, depending on the role given to a specific doctor, set values for the treatment regimen for the specific patient.

Data stored on the medical device controller 4 may also be sent by a secure encrypted communication to a secure server such as a medical data server 6 of a hospital or healthcare practitioner, such that for instance the history of treatment is stored on a remote secure server and accessible by healthcare professionals. The secure medical data server 6 may also upload information to the medical device controller 4 such as a new treatment regimen or information for the Journal concerning user's roles and rights, including updates with new medical practitioners that should have access to the medical device 2.

A corruption of clinical parameters values in a journal in a consumer electronic device 12a, 12b, 12c, resulting in a corrupted journal, may represent a safety risk. However, once the corrupted electronic device is paired with the medical device controller 4, the content of the journal on in the medical device controller 4 and the corresponding journal in the electronic device are compared by the medical device controller and the latter generates an alarm if the content, in particular the journal entry history of said journals do not match.

In an embodiment, the medical data server keeps a safe and valid copy of the journals stored in the medical device controller in case the corrupted journal of a consumer electronic device cannot be verified by the medical device controller 4. A verification of the integrity of the journals stored in a consumer electronic device may therefore be done with the journals stored in the medical data server 6 when the medical device controller 4 is not available. Communications between the medical data server 6 and the medical device controller 4 may be achieved for example through a GSM, 4G, LTE or IOT communication network for direct communications or through a consumer electronic device 12 which is connected to the medical device controller for indirect communications.

Information from the secure medical data server 6 and the medical device controller 4 may also be used to provide information and alerts to the patient, for instance to announce a need for a medical visit. Such information may also include information for renewing or ordering a new drug container unit.

The medical device controller may further be paired and establish communication with medical device components such as the drug container, infusion set, analyte sensors, and other peripheral components forming part of the drug delivery system. Some of these medical components may be secure components and some may be non-secure components, the principle of secure connection between the non-secure or secure components being the same as described above.

The computer program of the medical device controller 4 is designed to perform in particular the following tasks: transmitting instructions to the software application installed on a consumer electronic device in order to upload from the catalogue of images stored in the memory of the consumer electronic device, an image of an appropriate user-interface template for data entry, or an image containing a particular message for display on the consumer electronic device; checking and validating parameters entered through the user-interface of a consumer electronic device; executing algorithms for data calculation and data conversion; data encryption and data transfer to a consumer electronic device and to the medical device 2 of the medical device and secure control system 1; verifying the integrity of each consumer electronic device in communication with the medical device controller 4 and the integrity of the medical device; and management of journal entries in journals stored in the memory of consumer electronic devices 12a, 12b, 12c, the medical device controller 4, and the medical device 2.

The computer program is configured to retrieve parameters contained in journals stored as files in the memory 14 of the medical device controller 4 and to execute different commands based on these parameters. The journals also ensure secure communications with the drug delivery device and with consumer electronic devices.

In an embodiment as shown in figure 5, the medical device 2 comprises a pump unit 20, a drug unit 22 containing a drug to be administered, a dosing unit or an infusion set 24. The pump unit 20 comprises a pump drive, a control system, and a power supply, for instance in the form of a battery. The control system of the pump unit comprises a processor, a communications module for communication with at least the medical device controller 4, and a memory for storing data including at least a Journal containing information related to control of the drug delivery device. In an embodiment, the pump unit may be in the form of a reusable (multi-use) part. The drug unit 22 is coupled to the pump unit and may be a disposable or consumable part that is replaced by a new drug unit for coupling to the pump unit 20 when the drug contained therein is consumed or after a certain time interval.

In a preferred embodiment, the drug unit 22 comprises a drug container mounted on a support that may advantageously be provided with an adhesive surface for mounting on a patient's skin. The drug container unit may further comprise a needle injection system with a cannula for subcutaneous administration of the drug. A drug delivery device according to embodiments of the invention may however have other configurations. In a first variant, the pump unit and drug container unit may form a single disposable unit. In another variant, a separate patch unit with a transcutaneous needle may be connected by a flexible tube to the drug container coupled to the pump unit. Other drug delivery device configurations within the scope of the present invention are possible in conjunction with various medical component peripheral devices. The drug delivery device may further comprise a one or more sensors useful for monitoring aspects of the disease or condition being treated, for instance blood analyte sensors such as blood sugar sensors for diabetes treatment, blood pressure and heart rate sensors, temperature sensor, and others.

The communications module of the pump unit control system comprises wireless communications transceivers, including at least short range wireless communications transceivers according to standard protocols such as Bluetooth for local communication with devices in the vicinity of the drug delivery device, in particular the medical device controller 4, and may further comprise other communication transceivers for mid-range communications such as WiFi, and optionally long range communications such as GSM or other cellular phone communication technology.

Drug delivery devices with a reusable pump unit coupled to a disposable drug unit for mounting against a patient's skin are *per se* generally known in the art. In conventional systems, such units are typically controlled directly on the reusable part, or by a separate controller that comprises a graphical user interface with a screen for inputting and displaying data to the patient.

As apparent from the above description, an advantageous feature of the invention resides in the journals. Data transfer between two devices is preferably in the form of the exchange of journal entries in order to carry out a particular command. Journals may also be used to perform several functions such as verifying the integrity of each device attempting to connect to the medical device controller 4, or assigning different rights to each user connected the medical device controller 4 via their consumer electronic device 12, such as the right to select a new bolus value for insulin injection within a range preset by a doctor.

Journal entries contain all the data necessary to operate the medical device according to any particular application. The difference between using data in a journal entry compare to data stored in variables is that the journal keeps the traceability of data modification: chronology, time and geo-localization information, identity of the user and the consumer electronic device 12 requiring the data modification. Using Journals instead of variables have several advantages:
1. Data modification could be placed in the Journal as a pending request, and the data modification could be treated and validated later, when all the data necessary for a specific activity are available which may be obtained from different sources (users, data server, peripheral, consumer electronic device). For example, a user could start the process of pairing a second consumer electronic device 12. The new journal entry will be validated once the doctor writes a journal entry that validates the new journal entry corresponding to the pairing of the second consumer electronic device.
2. Journal entry allows the management of multiple consumer electronic devices 12 connected to one medical device controller 4, or multiple medical device controllers connected to one consumer electronic device 12 because it is possible to define management policy and rules, for example to inform the consumer electronic device 12a that the consumer electronic device 12b has requested a data modification, and request additional confirmation from the users before validating the new data.
3. Journal entry allows each consumer device 12 to work off-line, i.e. neither connected to the medical device controller 4 nor to the data server. This is useful for example to allow the user to see the history of data (glucose level, insulin dose), and to introduce new data (food consumption, physical activity). If a decision is taken based on these data, when the consumer electronic device 12 is connected with the medical device controller 4, the journals will be compared, and the user could be informed that the journal in his consumer electronic device 12 are either obsolete or corrupted. The medical device controller 4 may require a confirmation from the user that the new data introduced off-line are to be taken in consideration and enter as a journal entry in the journal of the medical device controller.
4. The content of the journal could be used to validate user decision according to the history of the data to detect abnormal value, for example a bolus request higher than usual, taking into account the historical value in other journals as glucose value, insulin dose, time, location. If an abnormal value is detected, additional confirmation will be requested from the user before validating the journal entry and process it.

Different journals are created according to the type of data exchanged between the medical device controller 4 and any other devices of the medical device and secure control system 1 in order to simplify the management of the journals. All data exchanged between two devices are in the form of an exchange of journal entries. For each data transfer corresponding to a particular operation: i) between any consumer electronic device 12 and the medical device controller 4; ii) between one consumer electronic device and another consumer electronic device, or iii) between the medical device controller 4 and the medical device 2, a journal entry is written in the corresponding journal of the two devices between which a data transfer as occurred for a particular operation. Thus, the corresponding journal of both devices comprises an identical journal entry in the form of a list of values corresponding to this particular operation. A journal entry comprises a header corresponding to the journal in which the journal entry is written. The header is followed by a list of journal entry parameters which relates to the data exchanged between two devices according to the configuration i), ii) or iii) as set forth above. A specific operation between two devices triggers an exchange of journal entries between these two devices in order to update their respective journals. The exchange of journal entries between two devices implies that the content of one or more journal entries is placed in a file that is encrypted with a standard protocol such as Secure Shell (SSH) and the integrity of the file is secured with a HASH value added to the file.

A format of a journal entry is given below as an example.

### Journal Entry Header:

| | |
|---|---|
| *Type of activity:* | *reference value indicative of the journal type.* |
| *Journal Entry ID:* | *incremented at each transaction* |
| *Time:* | *time of the transaction, including time zone information for the management of time zone change.* |
| *Geo-localisation:* | *add a safety level for the validation of the journal entry* |
| *RX_ID:* | *ID of the Receiver* |
| *TX_ID:* | *ID of the Transmitter* |
| *Target_ID:* | *ID of the final Receiver, for forwarding Journal entry, for example from the medical device controller to the data server through the communication channel of the consumer product.* |
| *Validation ID:* | *incremented after each entry validation by the medical device controller* |

Non-limiting examples of journals stored in the medical device controller 4, the consumer electronic device 12 and the medical device 2 are provided hereafter.

### Pairing journal

The pairing journal contains parameters that are used to link the medical device controller 4 with any consumer electronic device 12a, 12b, 12c, the medical device 2, and a medical data server 6 in order to ensure a first level of secure connection for the medical device and secure control system. Two consumer electronic devices may also be paired together, for example a patient's smartphone 12a with a doctor smartphone 12b or a friend smartphone 12c.

A pairing journal may contain one or more of the following parameters: component type (e.g. smartphone, computer, patch pump, medical data server, medical device controller), medical device controller ID, pairing ID, user ID, serial number, Unique Device Identifier (UDI), device identifier: International Mobile Equipment Identity (IMEI), Sim card identifier: International Mobile Subscriber Identity (IMSI), Secure Shell (SSH) private and public keys, passwords, media access control address (MAC address). During a pairing operation between two devices, pairing parameters are exchanged between these two devices as a journal entry in the pairing journal stored in each pairing device and are compared together for identification.

Different pairing configurations between several devices involved in the medical device and secure control system may be applied according to the level of security required.

### User journal

A user journal contains parameters of all users added to the medical device and secure control system. Each user journal may contain one or more of the following parameters: user ID, name, phone, mail, password, GPS coordinates, active/inactive, user rights for each journal, and alarm settings.

### Session journal

A session may be defined as the initialization of the communication process between the medical device controller 4 and any one of a consumer electronic device 12, the medical device 2 and the medical data server 6. The session is open through an initialization of the communication with password, keys exchanged and verification of the pairing parameters. A session journal contains parameters related to the status of the session such as: session open, session valid/not valid, and session close.

### Safeguards parameters journal/ doctor clinical parameters journal

A safeguards parameters journal (doctor clinical parameters journal) contains safeguards parameters set by the doctor so as to ensure that the user cannot perform certain functions which may have life-threatening consequences. For example, the safeguards parameters may prevent a user to enter a bolus value for insulin injection which is outside a specific range. The safeguards parameters journal may contain one or more of the following parameters: target blood glucose, high blood glucose alarm level, low blood glucose alarm level, maximal basal flow rate, and maximal bolus value.

### Patient clinical parameters journal

A patient clinical parameters journal contains clinical parameters set by the patient such as basal curve (time window + flow rate), basal flow rate increment, bolus flow rate, bolus volume increment and low insulin level alarm.

### Components journal

A components journal contains information on all devices involved in the medical device and secure control system such as device ID, device type, device serial number, device hardware version and device software version.

### Consumable journal

A consumable journal may contain values obtained for example from a QR code, a RFID tag and a lot number and may also contain parameters such as consumable type, consumable batch or lot and consumable expiry date.

### Alarm journal

The alarm journal contains alarm settings that may be configured by a user and may include the following parameters: alarm type: low insulin, low battery, no cartridge, occlusion in the medical device; alarm sending modes: SMS, e-mail, image.

### Image journal

An image journal contain parameters to instruct the software application installed in any consumer electronic device 12 to upload from the catalogue of images stored in its memory an image of an appropriate user-interface template for data entry or an image containing a particular message for display on the consumer electronic device 12.

Each type of journal listed above is stored in the memory of each device which is part of the medical device and secure control system 1, in particular in the memory of one or more consumer electronic devices 12, in the memory of the medical device controller 4, in the memory of the medical device 2 and in the memory of the medical data server 6.

Journals are used to provide instructions from the medical device controller 4 to all devices in communication therewith and to add a second level of security to the first level of security achieved through the pairing operation as described above. This second level of security is performed by the medical device controller 4 which controls whether the journal entry history of all journals stored in the memory of any external device trying to connect with the medical device controller 4 in an attempt to control a particular operation, such as a bolus injection, correspond to the journal entry history of all its journals. The medical device controller 4 denies a login session for any external electronic device attempting to connect with it if the journal entry history of all journals of the external device does not correspond to the journal entry history of all the journals of the medical device controller 4. If both of these journal histories match, the medical device controller 4 allows a login session with the external device (e.g. consumer electronic device such as a smartphone) and updates their respective journal to add a login session entry.

One or more journals may be linked to other journals for certain operations. For example for a pairing operation between two devices, the pairing journal may be used in association with the component journal to retrieve more specific information on a component type that may be used during the pairing operation.

All instructions related to operating a medical device for a particular application are processed by using and modifying the information kept in a journal. The policy to add new journal entries in the journal is dependent on the safety level of concern of the instructions processed. The policy is based on two requirements:
1. User Role, given for each user, which defines which activities this user may perform, and
2. *"Trust Loop"* which defines how many users must be involved to perform an activity.

Because each user uses a device that includes a copy of the journal, then the *"Trust Loop"* defines how many journals must be modified for a specific activity. Because all journals can be compared between them, this creates a "loop" of comparison, i.e. the "Trust Loop". The more journals involved, the more secure is the control of the activity, because to enter journal entries necessary to execute this activity, it is necessary to access the journals of all devices involved in a particular activity.

The medical device controller 4 always maintain the journals that include all the information necessary to manage the medical application process, then the medical device controller 4 also are in charge to verifying that all journal required by a specific *"Trust Loop"* are identical.

To improve the *"Trust Loop",* it is also possible to give to a User a medical device controller 4 with only the functionality of maintaining a copy of the journal and providing means of communications. Then the presence of this device will be necessary to achieve a "Trust Loop". Such device can be given to the Doctor in charge of the patient, to the nurse to ensure that if several nurses are defined as user, only one at the time can enter journal entries, to user allowed to remotely enter journal entries which could be considered as a critical task.

To start a session between two paired devices, knowledge of the journal history of each device of the medical device and secure control system is necessary, including the journal history of one or more devices not involved in the session between these two paired devices, but present in the journal of one or both of these two paired devices because of a login session occurred between one or more extra devices and one or the two paired devices at different times in the past. If a journal entry is exchanged between two paired devices for data transfer corresponding to a particular operation in the absence of the medical device controller 4, the latter may access each journal of the two paired devices to validate this journal entry according to the *"Trust Loop"* requirements defined above. Only if these requirements are met, the medical device controller 4 validates the journal entry and writes in the header of the journal entry a confirmation that the journal entry has been validated.

A journal entry which has not been validated by the medical device controller 4 is considered as a pending entry. If two journals of two paired devices do not match, the medical device controller 4 may request from the users involved in the *"Trust Loop"* to enter the missing journal entry, and may require an acknowledgment from these users. When all the conditions required by the *"Trust Loop"* are met, the journal entry is validated, and the related information can be used for operating the medical device according to a particular application.

The medical device controller 4 may free its memory by archiving journal entries in the medical data server 6 that are not relevant for the operation of the medical device, and may instruct other devices of the medical device and secure control system 1 to do the same.

The session being dependent of the journal content, the medical device and secure control system is protected. If for example the patient electronic device 12a (e.g. smartphone) is stolen, the patient may get an alarm from the medical device controller 4 and the medical device 2. Then the patient can use an emergency mode to connect to the medical device controller. This process will add a new entry in the session journal. Connecting the stolen smartphone will not work because the journal in the stolen smartphone will not match the journal of the medical device controller. To recover the smartphone a "trust loop" must be created that involves a third trusted device such as a family user smartphone, a smartphone connected in emergency mode, or a medical data server. Copying a journal entry stolen during a communication and introducing it in the communication to force an unwanted operation will not work because each journal entry is different, being dependent on the history of the journal entries. Altering a value entered by the user will not work either, because the journal entry corresponding to a particular operation includes information from the preceding journal entry. Both entries have to match together, the one before the user enters a value, and the one with the value entered by the user.

### Initialization of the medical device controller

Once the hardware manufacturing of the medical device controller 4 is completed at the manufacturer site, operating system software may be installed in the medical device controller 4 for operation thereof. Empty files are downloaded in the memory 14 of the medical device controller 4 for the creation of different journals. Journal entries will be incrementally written in the corresponding journals during the life cycle of the medical device controller 4.

Two identical pairing journals containing pairing parameters as described above may be stored in the memory of the medical device controller 4 and in the medical data server 6 at the manufacturer site. In an embodiment, the pairing parameters are entered in a pairing journal stored in the medical data server 6. The medical device controller 4 is then connected to the medical data server 6 through a communication network, for example an internet of things (IOT) communication network, and a copy of the pairing journal is stored in the memory of the medical device controller 4. Journal entries about a medical practitioner (medical doctor) that will be responsible for the creation of further journal entries that will link a patient electronic device 12a with the medical device controller 4 are also entered in the corresponding journals of the medical data server 6 and the medical device controller 4.

The medical doctor may access the journals stored in the medical data server 6 through for example an htpps protocol for secure communication after having entered in his computer 12b a password obtained at the manufacturer site in order to add a new consumer electronic device.

### Adding a user by a medical practitioner (medical doctor)

Adding a patient in the medical device and secure control system is a critical process from a safety point of view. The *"Trust Loop"* involves the doctor, the patient, the medical data server 6, the medical device controller 4 and the associated journals. In order to add a patient to allow him to operate the medical device 2, through for example his smartphone, the medical doctor may open a session with the medical data server 6 and selects in the user journal a user to be added in the medical device and secure control system 1. A pairing operation is then performed between the medical device controller 4 and the medical data server 6, through for example an IOT communication network, during which pairing parameters are compared for identification of the medical device controller 4. The medical controller then updates its journals and the journals of the medical data server 6. The patient downloads the software application on his smartphone and uses the camera of the smartphone to capture an identification code on the medical device controller 4 which may for instance be in the form of a QR code. The identification code is then entered by the doctor on his computer 12b and sent to the medical data server 6. The doctor will then be able to enter a journal entry in the user journal stored in the medical data server 6 and assigns to the user different rights for particular actions the patient may perform with his smartphone 12. The medical device controller 4 then copy in its memory the user rights parameters through the communication network.

The medical device and secure control system according to several embodiments may be used in various situations as it will be apparent from the description hereafter of non-limiting examples as shown particularly in figures 5 to 11. Considering that the concept of journal entries for data exchanged between devices of the medical device and secure control system and for integrity verification of each external device in communication with the medical device controller 4, has been already discussed, journal entry in the corresponding journal of each device for each operation will not be described in the following examples for conciseness reasons.

### Example 1: Patient setting a new bolus

Referring to figure 5, a patient may need a bolus injection of insulin at a new dosage and may wish to set a new bolus value through his consumer electronic device 12a which may be for example a computer. To this end, the medical device controller 4 and the computer 12a are in communication to each other by Bluetooth. The computer 12a comprises a software application which has downloaded a catalogue of images in its memory during its installation. The catalogue of images comprises user-interface templates with one or more data entry fields for user input and images containing different messages, for example instructions. The user may request a login session with the medical device controller 4 via the software application. Journals stored in his computer comprise journal entries previously entered by his medical doctor in order to assign different rights to the user connected the medical device controller 4 such as the right to select a new bolus value for insulin injection within the range preset by the doctor. Upon successful pairing, the medical device controller 4 sends instructions to the computer 12 to upload from its memory a user-interface template for display on the computer screen with a data entry field for password input. A password is entered by the user and the computer captures at least a portion of the displayed image on the computer with the password once entered. A session is opened if no integrity breach has been detected based on identification or control pixels contained in the image. Upon identification, the medical device controller 4 transmits a new navigation menu to the computer 12, wherein the user may select the bolus menu. A screen capture of the bolus menu may then been transmitted to the medical device controller 4 which checks in its memory the user role and its associated rights corresponding to this particular action. The medical device controller 4 then controls whether the bolus value is within the allowed range set by the doctor and transmit the new value to the drug delivery device 2 (e.g. patch pump). The medical device controller stores in its memory and manages the encryption keys for secure communication with the drug delivery device.

During all the communication exchanges occurring between the medical device controller 4 and the computer 12 and between the drug delivery device 2 and the medical device controller 4, journal entries are created to ensure a so-called *"trust loop"* between all the devices and to maintain the traceability of the whole injection process.

### Example 2: Pairing of a new user

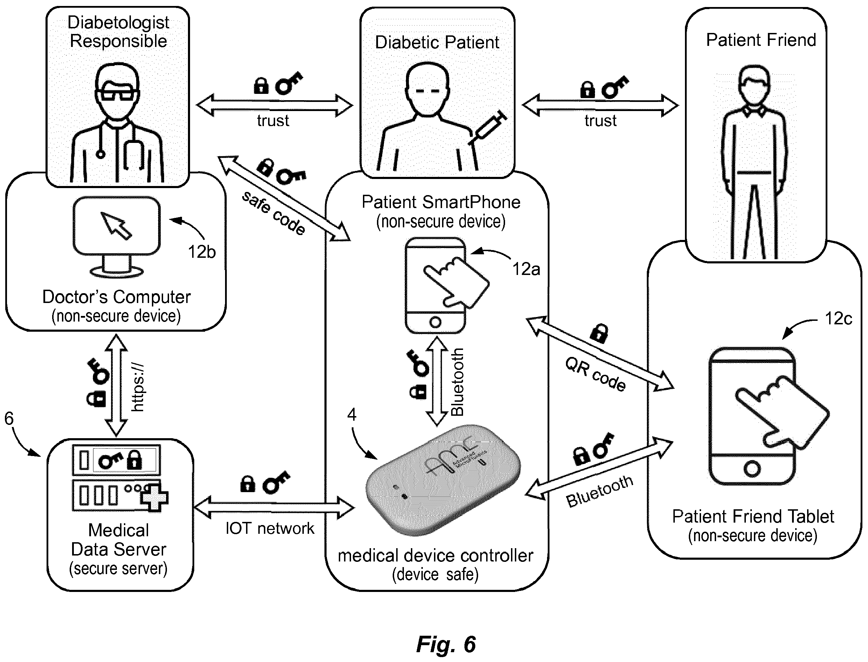

Referring to figure 6, a diabetic patient, in an exemplary situation, may want to add as a "backup" a friend as a new user with certain rights. Adding a new user must be done with a doctor who must "trust" the ability of a friend to manage the diabetic status and the insulin pump. To that end, the medical doctor establishes a *"secure device pairing"* between his consumer electronic device 12 (e.g. computer) and the medical data server 6 through a communications system, for example an https protocol communication. The medical doctor may request a login session through the medical data server 6. The doctor selects a patient and requests to add a user. The medical data server 6 opens a session with the medical device controller 4 through for example an IOT communication network after a pairing operation. The medical device controller 4 is then paired with the patient electronic device 12a (e.g. smartphone) and sends instructions thereto so as to open a session. The medical device controller 4 then sends instructions to the patient electronic device 12a to display on its screen a patient identification user-interface. Once the patient has been identified, the medical device controller 4 sends a request to the doctor's computer 12b for authentication. A safe code may be used to validate the connection between the medical device controller 4 and the patient electronic device 12a. To that end, the medical doctor gets the session Safe Code from his computer 12b. The doctor enters the Safe Code on his computer in order to connect the medical data server 6 and fills the new user form. The medical device controller sends instructions to the patient electronic device 12a to transmit a code, for instance a QR code, to a friend electronic device 12c. The friend opens a session and completes user/pairing parameters which are transmitted to the medical device controller. The medical device controller updates the journal of the friend computing device, confirms entry and closes session.

### Example 3: Patient changing consumable

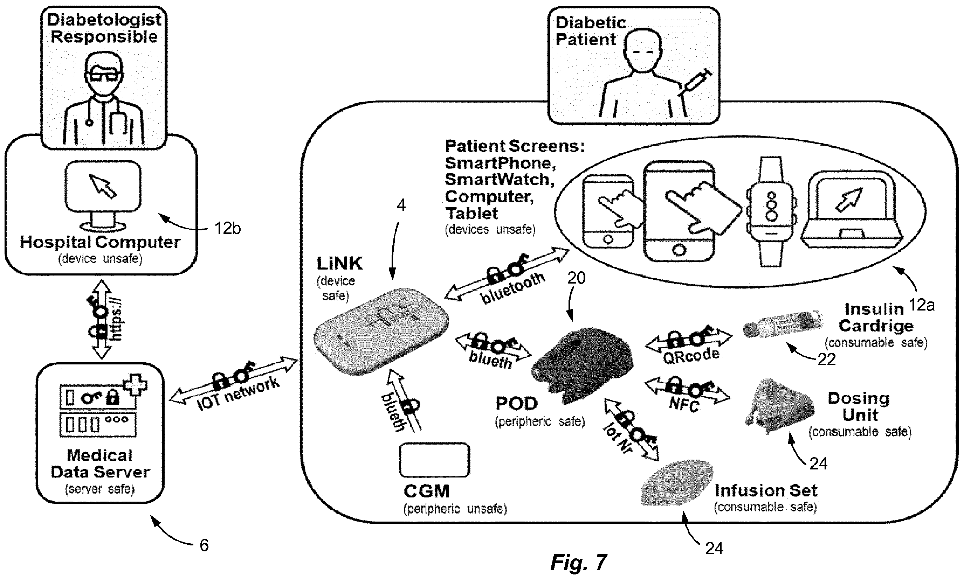

Referring to figure 7, the patient may need to change the consumable for insulin injection. The goal is to keep the traceability of the consumable used, comparing the consumables with the Clinical Journal, verifying the expiry date. The patient may use different possibilities to record the consumable data: Dosing Unit - Near Field Communication (NFC); Insulin Cartridge - QR Code; Infusion Set - LOT number entry.

The patient may start a software application on his consumer electronic device 12a to open a session. The patient may use for example a smartphone, a computer tablet, a smartwatch or a computer as a consumer electronic device 12a which is paired with the medical device controller 4 using for instance some of the pairing parameters discussed under the "pairing journal" section. The medical device controller 4 may transmit to the patient electronic device instructions to display on its screen an image input password for password entry. In an embodiment, the software application may capture for instance the image with the input password and may transmit this image to the medical device controller. In another embodiment, the software application may capture only a portion of the image with the input password and may transmit this portion of the image to the medical device controller.

The medical device controller may send instructions to the patient electronic device to open a session. For instance, the medical device controller 4 may send instructions to the patient electronic device 12a to display on its screen an image with the indication such as "Empty Cartridge" (relevant data of the image stored in a memory of the patient computing device). The patient may select a menu such as "exchange the cartridge". The patient electronic device may send the relevant information to the medical device controller 4 which in response may send instructions to the patient electronic device to display on its screen an image with an indication to detach the patch pump. The patient may then detach the patch pump and may select on his electronic device an action, for example "Next", such that the patient electronic device 12a may send the relevant information to the medical device controller 4 which in response may send instructions to the patient electronic device to display on its screen an image with indication such as "Enter infusion set lot No." and the selectable menu "Next", "info", "Cancel". The patient may enter the number of the lot and may select "Next". The patient electronic device 12a may send the relevant information to the medical device controller 4 which in response may send instructions to the patient electronic device to display on its screen an image with the indication such as "Scan dosing unit" and the selectable menus: "Scan", "info" and "Cancel".

The software application may then activate the NFC function of the patient electronic device to read a NFC tag from the dosing unit and the relevant data are transmitted by the patient electronic device to the medical device controller 4. Upon receipt of these data, the medical device controller may send instructions to the patient electronic device to display on its screen an image with an indication such as "Read QR code of insulin Cartridge" and the selectable menus: "Scan", "info" and "Cancel". The software application may activate the camera to read the QR code data from the insulin cartridge. The patient electronic device sends the relevant data to the medical device controller 4. The medical device controller 4 then sends instructions to the patient electronic device to display on its screen an image with an indication such as: "Please attach to the skin the patch pump with new consumable and the selectable menus: "Done", "info" and "Cancel" (relevant data of the image stored in a memory of the patient computing device). The patient may select the menu "info". The patient electronic device may display a short instructions movie. The patient may install the patch pump 2 according to the instructions and may select the menu "done". The medical device controller 4 may then start a session with the patch pump. The medical device controller may send instructions to the patient electronic device to display on its screen an image with indication such as: "Start priming the injection" and the selectable menu "Start", "Info" and "Cancel". The patient may select the menu "Start" and the patient electronic device may send to the medical device controller the corresponding instructions. The medical device controller may send instructions to the patient electronic device to display on its screen an image with an indication such as: "Priming running, please wait" and the menu: "Cancel". The medical device controller may start the priming process with the patch pump, keeps the journal traceability of the priming process until the process ends and closes the session with the patch pump. The medical device controller may then send instructions to the patient electronic device to display on its screen an image with an indication such as "Priming finished, device ready" and the selectable menu "Done" and "Exit"

The medical device controller 4 may then established an encrypted connection with the medical data server 6 through a pairing operation and send a copy of its journals which is saved in the medical data server. A medical practitioner my then access the patient's data through for example his computer 12b using a secure connection.

In parallel to the change of consumable, the reusable part of the pump as well as the medical device controller 4 shall be recharged. From a security standpoint, it is particularly appropriate to use a wireless power transfer using inductive charging, such as QI standard, in order to recharge these reusable parts.

A key advantage of the use of a wireless charging protocol, stems from the physical separation between the charger and the medical device 2. Wireless charging protocol may avoid several problems that may arise from a physical connection (using for example a USB cable and connector to recharge the medical device controller 4 and the medical device 2 which may be in the form of a drug delivery device). These problems may for example be: a poor power supply that can destroy the medical device via the USB cable; a USB connector that can make the medical device sensitive to electrostatic discharges; and a connector that is difficult to clean and that may cause sterility issues.

Accessories including QI wireless charger and cables which may be furnished with the medical device controller 4 and the medical device 2 comply with the medical device safety requirements. QI wireless charging protocol isolates the medical device from the consumer electronic device which is coherent with the medical device and secure control system, whereby the software of the medical device controller 4 is dissociated from the software of the consumer electronic device.

### Example 4: Nurse setting a remote injection to a disabled patient

Referring to figure 8, in an exemplary situation, a nurse may assist disabled patients for taking a meal. After the meal, the nurse may set an adapted bolus to the disabled patients. To this end, the nurse connects her tablet to the medical data server 6. For each disabled patient, she consults the patient data and history, and sets the appropriate bolus. Each patient will receive a message containing information regarding the bolus setting. The patient can validate the bolus setting according to his/her User Role/Rights.

More particularly, the nurse starts her application software on her phone 12c to open a session. The medical data server 6 sends instructions to the nurse phone to open the session and to display a menu, whereby different patients can be selected. The nurse selects a patient, reads the patient's data and updates food information and request basal/bolus advice. The data are sent to the medical data server 6 which opens a session with the medical device controller through an internet of thing (IOT) communication network. The medical data server 6 evaluates basal/bolus advice from clinical journal, data food, glucose levels, clinical patient insulin dose and clinical doctor setting. The medical data server 6 sends data to the nurse phone to display an image with basal and bolus value recommendations.

### Example 5: Runner using a Smartwatch to control glucose and inject insulin

Referring to figure 9, in an exemplary situation, the patient may practice running and may use a user interface that is adapted to his/her activities to manage glucose levels and insulin treatment. To this end, the patient may use a Smartwatch with an adapted and simplified interface. Continuous glucose monitoring (CGM) continuously updates the medical device controller with the Journal glucose value. In the present invention, a smartwatch shall be interpreted as a consumer electronic device held on a wrist, and that has at least a short range wireless communications capability for communication and that may run software applications.

### Example 6: Mother managing treatment for her diabetic daughter

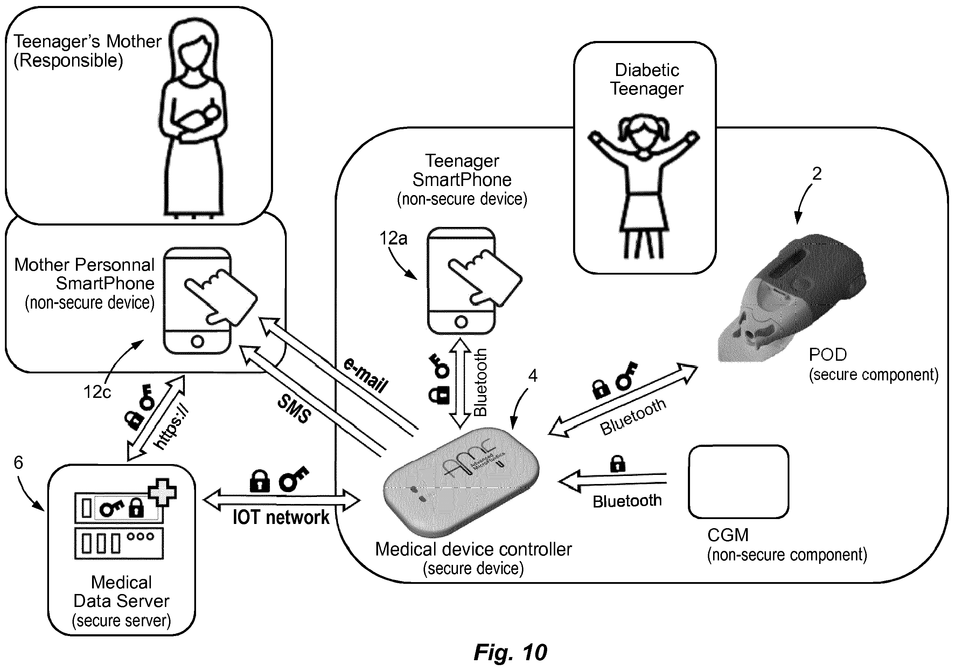

Referring to figure 10, a mother may remotely monitor the glucose level of her daughter ("patient with limited rights"). She may receive an alarm "glucose level" and react remotely by setting a bolus. The medical device controller continuously checks the glucose level, and sends an alarm according to the criteria set in the safeguards parameters journal and the alarm journal. Once the mother receives the alarm, she connects to the medical data server and decides with the clinical data of his daughter to set a bolus.

### Example 7: Obtaining the status of both the medical device controller and the patch pump from any device

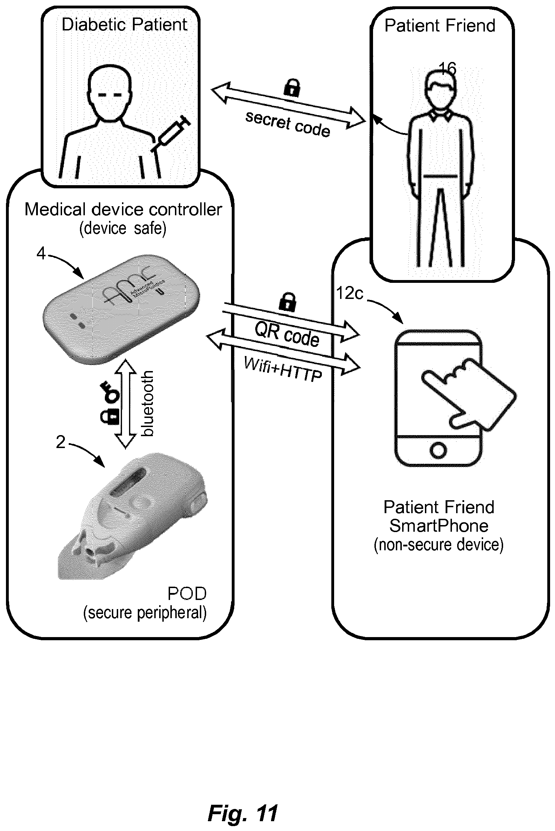

Referring to figure 11, patient's Smartphone may not be available (lost, battery low), and the patient may want to use the Smartphone 12c of a friend, not paired and without any application loaded, to retrieve information regarding, for example, the insulin pump status, the battery level, remaining insulin in the cartridge and glucose value. To this end, the patient may use an emergency mode to connect the friend's Smartphone to the medical device controller 4 via an HTTP WEB server available on the medical device controller. The Friend gets the connecting information to the medical device by capturing with his Smartphone 12 a QR Code placed on the medical device controller.

### List of referenced features:

**Medical device and secure control system 1**
**Medical device 2** (Secure device)
   Drug delivery device
   Pump [Reusable part] 20
      Pump drive
      **Control system**
         Processor
         Communications module
         Memory
            **Journal**
      Power supply (battery)
   Drug container [Single use part (disposable / consumable] 22
      Identification tag
      RFID, QR Code, Lot nr, identification nr
   Delivery components [Single use part (disposable / consumable] 24
      e.g. infusion set, patch pump disposable part, ...
**Medical device controller 4** (Secure controller device)
   Memory 14
   Processor 15
      Journal 16
      Operating parameter lookup tables
         Threshold settings
   Communications module 17
      Bluetooth
      WiFi
      IOT
   Limited user interface 18
**Medical device peripheral component 5**
   e.g. sensor (e.g. glucose sensor)
**Medical data server 6**
**Data communications network 8**
   Global communications network (internet)
   Local network
   IOT network
**Application specific devices 10**
**External electronic devices (generic devices / consumer electronic devices) 12**
   Personal Computing device (smartphone, portable computer, digital watch, ...)
   User interface
   Display screen
**Users**
   Patient
   Health care professional
      Doctor
      Nurse
   Non-professional
      Responsible
      Family
      Friend
Secure device
Non-secure device
Secure component (peripheral)
Non-secure component (peripheral)
Secure server
Pump [Reusable part] 20
   Pump drive
   **Control system**
      Processor
      Communications module
      Memory
         **Journal**
   Power supply (battery)
      Drug container [Single use part (disposable / consumable] 22
      Identification tag
      RFID, QR Code, Lot nr, identification nr
      Delivery components [Single use part (disposable / consumable] 24
      e.g. infusion set, patch pump disposable part, ...

## Claims

1. **Method** of operating a medical device and secure control system (1) comprising a medical device (2) and a medical device controller (4), the medical device controller comprising a communications module (17) including at least a short range wireless communications capability for communication with one or a plurality of consumer electronic devices (12) including any one of a smartphone, a personal computer, a smartwatch and a computer tablet, wherein each of the medical device (2) and the medical device controller (4) comprises a memory storing one or a plurality of journals (16) for exchange of journal entries between the medical device controller (4) and the medical device (2), and between said medical device controller (4) and said one or a plurality of consumer electronic devices, the method comprising:
a) receiving in the medical device controller (4) pairing parameters from a consumer electronic device (12);
b) comparing these pairing parameters with pairing parameters stored in the memory (14) of the medical device controller (4) during a pairing operation;
c) receiving in the medical device controller (4) a journal entries history of said consumer electronic device (12) in case of a successful pairing operation;
d) comparing the journal entries history received from said consumer electronic device (12) with the journal entries history stored in the memory (14) of the medical device controller (4);
e) validating a session between said consumer electronic device (12) and the medical device controller (4) for data exchange if the journal entries history of said consumer electronic device matches the journal entries history of the medical device controller.

2. **Method** according to claim 1, further comprising writing a journal entry in the at least one journal of the medical device, in the at least one journal of the medical device (2), and in the at least one journal of said consumer electronic device (12), preferably through a communications network (8) such as an internet of thing (IOT) network, wherein the journal entry is indicative of the type of data exchange under step e).

3. **Method** according to claim 1, wherein the medical device controller (4) is configured for communication with at least a first and a second consumer electronic device (12a, 12b), the method further comprising writing a journal entry in the at least one journal of the medical device, in the at least one journal of the medical device (2), in the at least one journal of said first consumer electronic device (12a), and in the at least one journal of said second consumer electronic device (12b).

4. **Method** according to claim 1 or 2, wherein, once the session under step e) has been validated, the method further comprising:
- sending instructions from the medical device controller (4) to said consumer electronic device (12) to display an image with one or more entry field for data input; and
- receiving in the medical device controller (4) a copy of the image or at least a portion of the image, sent by said consumer electronic device, comprising data input for operation of the medical device.

5. **Method** according to the preceding claim, wherein said image or said at least a portion of the image contains pixels that constitute an identification code which is read by the medical device controller (4) upon receipt of the image so as to determine whether the image displayed on said consumer electronic device (12) corresponds to the image sent from said consumer electronic device to the medical device controller (4) in order to detect a spoofed or hacked transmission of information.

6. **Method** according to claim 4 or 5, further comprising:
- receiving in the medical device controller (4) pairing parameters from the medical device (2);
- comparing these pairing parameters with pairing parameters stored in the memory (14) of the medical device controller (4) during a pairing operation;
- receiving in the medical device controller (4) a journal entries history of the medical device (2) in case of a successful pairing operation;
- comparing the journal entries history of the medical device (2) with the journal entries history stored in the memory (14) of the medical device controller (4);
- validating a session between the medical device (12) and the medical device controller (4) if the journal entries history of the medical device (2) matches the journal entries history of the medical device controller (4), and
- operating the medical device based on said data input.

7. **Medical device and secure control system** (1) comprising a wearable medical device (2) and a medical device controller (4), the medical device controller (4) comprising a communications module (17) including at least a short range wireless communications capability for communication with a consumer electronic device (12) including any one or more of a smartphone, a personal computer, a smartwatch and a computer tablet, the medical device controller (4) further comprising a memory (14) storing a computer program and encryption keys configured to establish an encrypted communication with the consumer electronics device, and a processor (15) configured to execute the computer program, the medical device controller (4) and the medical device (2) storing each at least one journal (16) for exchange of journal entries between the medical device controller (4) and the consumer electronic device and between the medical device controller (4) and the medical device (2), the journal entries comprising user identification, user rights, and operations instructed to the medical device, the medical device controller (4) configured to transmit via said encrypted communication:
i) a data entry image for presentation on a graphical display of the consumer electronic device, or
ii) instructions to upload an entry image stored in the memory of the consumer electronic device, and to receive via said encrypted communication an instruction for operation of the medical device,
the medical device controller (4) configured to transmit said instruction for operation of the medical device to the medical device.

8. **Medical device and secure control system** (1) according to the preceding claim, wherein the medical device controller (4) is a portable medical device controller formed as a separate component from the wearable medical device (2) and intended to be carried separately in short range wireless communication with the wearable medical device, the medical device (2) comprising a control system comprising a communications module including at least short range wireless communications capability, the communications module (17) of the medical device controller (4) including at least a short range wireless communications capability for communication with the medical device (2).

9. Medical device and secure control system (1) according to claim 7, wherein the medical device controller (4) is incorporated or physically connected to the medical device (2).

10. Medical device and secure control system (1) according to any preceding claim, wherein the at least one journal (16) stored in the medical device controller (4), the consumer electronic device (12) and the medical device (2) comprise each past instructions forming a journal entries history exchanged between the medical device controller (4) and the consumer electronic device (12) and between the medical device controller (4) and the medical device (2), the medical device controller (4) configured to verify the integrity of the consumer electronic device based on the comparison of both journal entries histories stored respectively in the medical device controller (4) and the consumer electronic device (2).

11. Medical device and secure control system (1) according to the preceding claim, wherein a login session between the consumer electronic device and the medical device controller (4) is allowed only if both journal histories are identical.

12. Medical device and secure control system (1) according to the preceding claim, wherein the medical device controller (4) is configured to incrementally write an entry in the at least one journal (16) stored in its memory for each login session with the consumer electronic device (12) and with the medical device (2), the medical device controller (4) further configured to copy said entry in the at least one journal stored in the memory of the consumer electronic device and in the memory of the medical device (2).

13. Medical device and secure control system (1) according to any preceding claim, further comprising a medical data server (6) storing a copy of the at least one journal stored in the medical device controller (4), and secure communications between the medical data server (6) and the medical device controller (4), wherein the medical device controller is configured to update said copy through said secure communications.

14. Medical device and secure control system (1), according to the preceding claim, wherein the medical data server (6) is configured to verify the integrity of the at least one journal stored in the consumer electronic device (12) when the medical device controller (4) is not available.

15. Medical device and secure control system (1) according to any preceding claim, wherein the medical device is a liquid drug delivery system, preferably a patch pump.
